# EUROPEAN PATENT SPECIFICATION

(11) **EP 1 808 133 B1**
(45) Date of publication and mention of the grant of the patent: **12.11.2008**
(21) Application number: 07006807.7
(22) Date of filing: 28.09.1999
(51) Int. Cl.: A61B 17/02

(54) **Spinal disc space distractor**
Bandscheiben-Zwischenraumdistraktor
Écarteur de l'espace intervertébral

(30) Priority: 02.10.1998 US 102669 P
(43) Date of publication of application: 18.07.2007
(62) Divisional of application: 99944216.3
(73) Proprietor: Synthes GmbH, 4436 Oberdorf (CH)
(72) Inventor: Aebi, Max, Québec H2V 4P1 (CA); Steffen, Thomas, Montreal Qc, Canada H2Y 2B7 (CA); Schenk, Beat, 4536 Attiswil (CH); Paul, David, Phoenixville PA 19460 (US); Cottle, William, Vancouver, British Colombia (US)
(74) Representative: Lusuardi, Werther

(56) References cited:
- WO-A-98/34552
- US-A- 3 916 907
- US-A- 4 898 161
- US-A- 4 997 432
- US-A- 5 019 081
- US-A- 5 059 194
- US-A- 5 213 112
- US-A- 5 368 596

## Description

The present invention relates to a device and method for spreading apart adjacent vertebrae of a vertebral column so that an implant may be inserted therebetween. More particularly, the present invention relates to a distractor device shaped and configured for minimally invasive insertion and use, such as for distraction of vertebrae using an anterior or anterolateral approach.

Back pain can be caused by either one or a combination of the following: a loss of disc height, compression of nerve roots, degenerative disc disease, spondylolisthesis, etc. The current standard of treatment for people suffering from severe back pain requiring surgical intervention due to different types of pathology is by intervertebral fusion. Intervertebral fusion is achieved by fusing two adjacent vertebral bodies together by removing the affected disc and insertion of a suitably sized implant that allows for bone to grow between the two vertebral bodies bridging the gap left by the disc removal. Typically, the intervertebral fusion procedure involves the steps of removing a portion or all of the affected disc material, spreading apart adjacent vertebrae with a distractor, and inserting an implant into the space previously occupied by the removed disc material. This procedure can be done either from the front of the patient (anterior interbody fusion) or from the back (posterior interbody fusion). If done from the front, it is important to reduce the size of the distractor so that the procedure is as minimally invasive as possible and thus minimally interferes with and traumatizes the organs and vasculature between the vertebral region being treated and the insertion point.

Current implants used for interbody fusion include allograft rings/dowels and cages such as threaded cages. However, the technique for the insertion of these implants generally does not achieve distraction because of their height limitations, thus making it difficult to restore the natural disc height. The force necessary to insert such implants (such as a result of drilling and tapping) may cause damage to the vertebrae or vertebral endplates at the insertion site. Moreover, allograft products and cages made out of other brittle materials (e.g., carbon fiber and ceramics) may break during insertion, particularly when distraction is not used and external force is necessary to insert the implant. Threaded cages on the other hand do not restore lordosis, and do not allow for atraumatic distraction to restore disc height. Thus, there remains a need for improvements in this area.

A surgical instrument set for the insertion of intervertebral endoprostheses is known from US 4,997,432 KELLER. This known surgical instrument comprises spreading forceps that have, at their front end on each spreading jaw, an essentially U-shaped recess for supporting part of an implant. The arms for the U-shaped recess are angled relative to the longitudinal axis of the forceps. The spreading jaws and the arms remain essentially parallel and evenly spaced from each other when they are spread apart by the scissor-like articulation of the spreading forceps.

The two part form of appended claim 1 is based on this prior art.

In accordance with the principles of the present invention, a spinal disc distractor is provided to allow for an implant insertion technique to be performed during distraction of the disc space. The implants are slid into the disc space between the distractor blades, preferably while the blades are in contact with the upper and lower surfaces of the adjacent vertebral bodies. The distractor of the present invention is formed to be as minimally invasive and atraumatic as possible such that it may readily be used in an anterior or anterolateral approach. Thus, the distractor of the present invention is configured to be used in the confined spaces of the human anatomy through a small surgical incision and permits laparoscopic approaches like Balloon Assisted Endoscopic Retroperitoneal Gasless ("BERG") approach to be used.

In a preferred embodiment, the distractor of the present invention has a scissor-like configuration with a pair of handles pivotally connected together. A distractor jaw is coupled to a distal end of each handle such that movement of the handles together draws the jaws apart to separate the vertebrae being treated. In an even more preferred embodiment, the jaws and handles are pivotally coupled together in a double-acting scissor-like configuration to further reduce the space required to move the jaws apart and thus further minimizes the invasiveness of the device and procedure.

Although the handles, jaws, and distractor mechanism of the present invention may all lie in the same plane, in order to facilitate visualization of the treatment site during distraction and insertion of an implant, at least handles may be angled away from the plane of the distractor jaws. In a preferred embodiment, the distractor mechanism is angled downward with respect to the jaws and the handles are angled downward with respect to the distractor mechanism to further enhance visualization and also to permit greater space for the implant holder adjacent the proximal end of the distractor during insertion of the implant.

A locking mechanism preferably is provided adjacent to or in the handle to maintain distraction. The locking mechanism may include a spindle mounted on a first handle and passing through the second handle. An internally threaded speed nut is rotatably mounted on the threaded bolt such that movement of the speed nut along the bolt selectively inhibits movement of the second handle away from the first handle and thus maintains the vertebrae at the desired distracted position.

The blades of the distractor of the present invention are configured to increase versatility of the distractor. The blades are removably coupled to the distractor jaws. Thus, the blades may be changed, as necessary or desired, for a given procedure or patient.

The spinal disc distractor of the present invention is thus designed to distract disc space atraumatically with respect to both the vertebrae and the implant during endplate preparation, implant sizing, and implant insertion. The distractor may be used in a straight anterior, anterolateral, or lateral approach, and may be used in either an open or a laparoscopic procedure. Moreover, the distractor is designed to ensure the selection of an anatomically correct implant size by permitting the annulus to be fully stretched so that the largest possible implant may be inserted and compressed upon release of the vertebrae, thereby enhancing stability and assuring correct placement of the implant. Thus, the present invention permits disc height and lordosis to be restored. The jaws are shaped and configured to preserve the endplate and the vertebral body during distraction, as well as to permit insertion of an implant during distraction. The risk of breakage of allograft implants and other cages made from brittle materials during insertion is thereby reduced.

The detailed description will be better understood in conjunction with the accompanying drawings, wherein like reference characters represent like elements, as follows:
Fig. 1 is top elevational view of a distractor with removable blades;
Fig. 1A is a top elevational view of a distractor similar to that of Fig. 1;
Fig. 2 is a side elevational view of the distractor of Fig. 1;
Fig. 2A is a side elevational view of a distractor similar to that of Fig. 1;
Fig. 3 is a top elevational view of the distractor of Fig. 1 in a working configuration with the jaws separated for distraction;
Fig. 4 is a perspective view of a femoral ring implant which may be used with a distractor formed in accordance with the principles of the present invention;
Fig. 5 is a perspective view of a cage implant which may be used with a distractor formed in accordance with the principles of the present invention;
Fig. 6 is an isolated top elevational view of the blade of the distractor of Fig. 1;
Fig. 7 is a perspective exploded view of the distractor of Fig. 1;
Fig. 8 is a side elevational view of the blade of the distractor of Fig. 1;
Fig. 9 is a side elevational view partially in cross-section of the distractor of Fig. 1 with the blade removed;
Fig. 10 is a schematic illustration of the distractor of Fig. 1 with blades of different lengths in use, according to the presently claimed invention;
Fig. 11 is a schematic illustration of the distractor of Fig. 1 with angled blades in use;

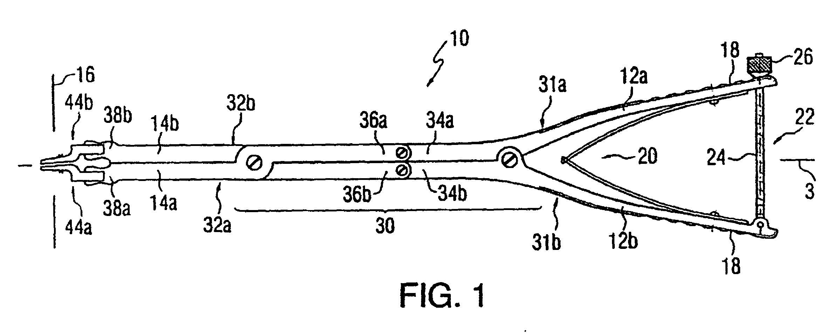

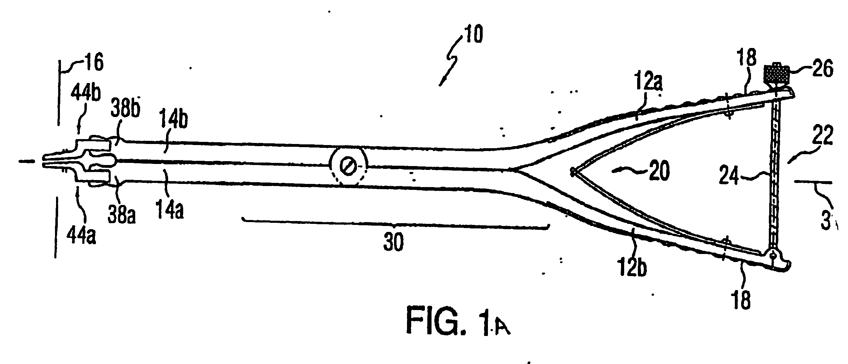

In accordance with the principles of the present invention, a distractor 10 is provided with a pair of handles 12a, 12b movable with respect to each other to actuate a pair of jaws 14a, 14b coupled thereto, as shown in Fig. 1. Although distractor 10 may be used for a variety of procedures, a preferred procedure for which distractor 10 is used is spinal disc distraction. Thus, distractor 10 is preferably configured such that actuation of handles 12 (12a, 12b) moves jaws 14 (14a, 14b) apart substantially along distraction axis 16 to a working position corresponding to the desired resulting relative position of the endplates. For example, the blades may be moved to a substantially parallel or lordotic position to separate adjacent vertebrae to be treated.

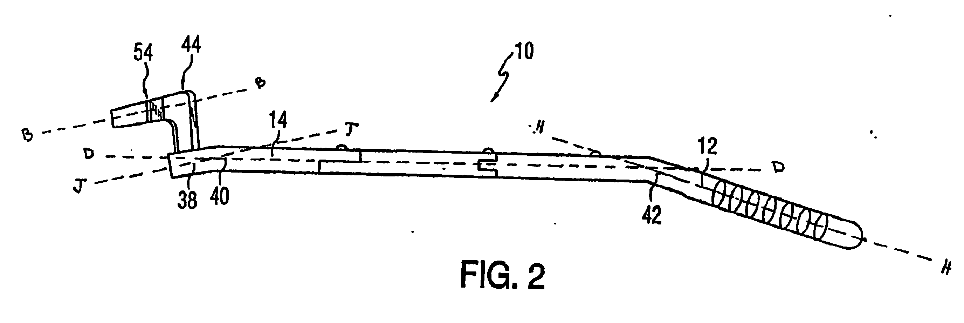

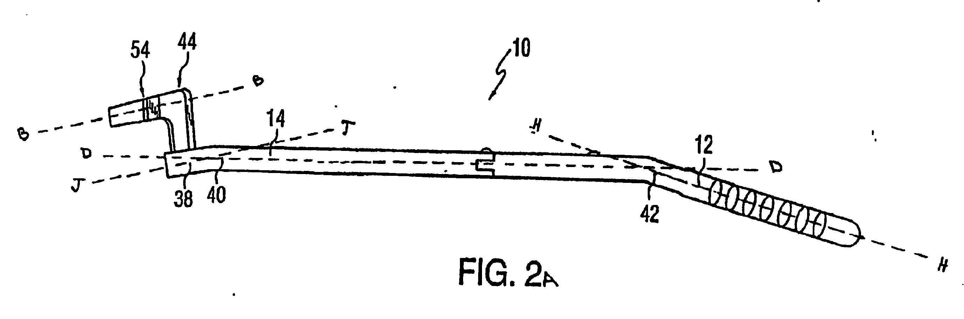

In order to be optimally useful for use in an anterior approach, handles 12 and jaws 14 are configured to move jaws 14 apart along distraction axis 16 a sufficient amount to adequately separate adjacent vertebrae to be treated (typically 5mm - 20mm, most typically 13mm - 15mm) yet to occupy a minimal amount of space within the insertion region during the procedure. Thus, handles 12 and jaws 14 preferably are pivotally coupled together in a scissors configuration such that movement of handles 12a and 12b together causes jaws 14a and 14b to move apart and effect distraction of vertebrae between which jaws 14 are positioned. Thus, proximal ends 18 of handles 12 are configured to facilitate gripping. In a preferred embodiment, the outer surface of handle proximal ends 18 is contoured to increase user comfort, as shown in Fig. 2.

A biasing element 20, such as a pair of leaf springs, maintains handles 12a, 12b in a spaced apart configuration such that jaws 14a, 14b are close together, ready for insertion through a small incision and narrow passage through the patient in the neutral configuration of Fig. 1. A locking mechanism 22 is provided to counteract biasing element 20 as desired, such as to maintain jaws 14 at a desired spaced apart position for operation on the distracted vertebral region. Locking element may be in any desired configuration, such as a threaded bolt 24 coupled (typically pivotally) to one handle and slidably passing through the other handle, and a locking nut 26 threadedly and rotatably mounted on the end of bolt 24 extending past the other handle (i.e., the portion not between handles 14). Movement of nut 26, as a result of rotation, along bolt 24 thus shortens the length of bolt 24 between handles 14 and prevents the handles from moving apart, thus maintaining handles 14 in a position closer together than the neutral position.

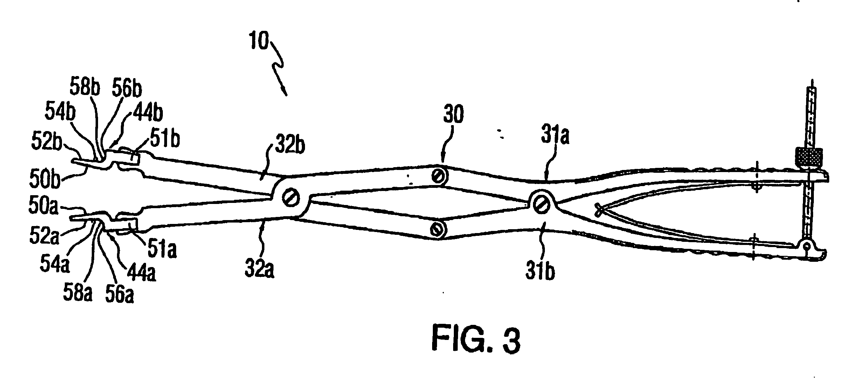

A distractor mechanism 30 is provided such that movement of handles 12 to actuate distractor mechanism 30 causes jaws 14 to move apart to effect distraction of adjacent element such as vertebrae. Distractor mechanism 30 may be have a simple scissors configuration (such as in Figs. 1A; 14-16 described below) such that handle 12a and jaw 14a are at opposite ends of a first lever arm and handle 12b and jaw 14b are on opposite ends of a second lever arm pivotally coupled to the first lever arm. In a preferred embodiment, distractor mechanism 30 is in the form of a double-acting scissor configuration having greater than one pivot point, thus reducing the amount of space required along distraction axis 16 and laterally away from distractor mechanism longitudinal axis 3 to effectuate distraction. As may be appreciated with reference to Figs. 1 and 3, in order to form a double-acting scissor configuration, handles 12 and jaws 14 are provided on separate lever arms which are pivotally coupled together. In particular, handle 12a is formed at a proximal end of proximal lever arm 31a, handle 12b is formed at a proximal end of lever arm 31 b, jaw 14a is formed at a distal end of distal lever arm 32a, and jaw 14b is formed at a distal end of distal lever arm 32b. Distal end 34a of proximal lever arm 31 a is pivotally coupled to proximal end 36a of distal lever arm 32a and distal end 34b of proximal lever arm 31 b is pivotally coupled to proximal end 36b of distal lever arm 32b. In order to actuate the double-acting mechanism to effectuate distraction and hence movement of jaws 14a, 14b apart upon movement of handles 12a, 12b together, one set of lever arms is laterally pivotally coupled together and the other set of lever arms is crosswise pivotally coupled together. In distractor mechanism 30 of Figs. 1 and 3, proximal lever arms 31 a, 31 b are laterally pivotally coupled together and distal lever arms 32a, 32b are crossed over each other and pivotally coupled together. However, it will be appreciated that, instead, proximal lever arms 31 a, 31 b may be crossed-over each other and distal lever arms 32a, 32b may be laterally pivotally coupled. The double-acting configuration breaks the pivoting action into two components, reducing the total movement of distractor mechanism 30 required along distraction axis 16.

An additional feature of distractor 10 which facilitates use thereof during distraction is the relative offset positions of jaws 14a, 14b, handles 12a, 12b, and distractor mechanism 30 with respect to one another, as may be appreciated in the side elevational view of Fig. 2. In particular, in a preferred embodiment, distal jaw ends 38a, 38b are to be positioned to properly distract adjacent vertebrae and distractor mechanism 30 and handles 12a, 12b are offset relative to distal jaw ends 38a, 38b to permit optimal visualization of distal jaw ends 38a, 38b from the proximal end of distractor 10 (outside the patient's body) during distraction. For example, a distal bend 40 may be provided immediately proximal of distal jaw ends 38a, 38b, as may be appreciated with reference to Fig. 2. Thus, the remainder of distractor 10 (i.e., the proximal portions of distractor 10 such as distractor mechanism 30 which lies in vertical plane HH shown in Fig. 2 and handles 12a, 12b which lie in vertical plane HH shown in Fig. 2) is in a different plane from distal jaw plane JJ of distal jaw ends 38a, 38b and the distraction site. With such an offset, visualization of the distraction site and of insertion of the implant therein is enhanced. Additionally or alternatively, a proximal bend 42 may be provided immediately distal of handles 12a, 12b such that proximal handle ends 18a, 18b lie in plane HH which is offset from distal jaw plane JJ of distal jaw ends 38a, 38b and the distraction site. The provision of either or both of bends 40, 42 causes at least a proximal portion of distractor 10 to be in a plane different from the plane of distal jaw ends 38a, 38b and the distraction site such that the line of site to view distraction is not obstructed by the distractor. Moreover, such offset of portions of distractor 10, such as distractor mechanism 30 and handles 12a, 12b, from the distal jaw ends 38 accommodate an implant holder for insertion of the implant to permit a substantially straight insertion of the implant holder. Bend 40 may be between 0°-30°, most preferably 10°, and bend 42 may be between 0°-30°, most preferably 15°, to achieve the desired improved visualization and increased area for the implant holder.

A distractor provided in accordance with the principles of the present invention is configured to distract adjacent vertebrae so that an implant may be inserted therebetween. Preferably, each jaw of a distractor formed in accordance with the principles of the present invention is provided with a blade shaped and configured to contact a vertebral endplate and also to permit insertion of an implant therebetween. Once the implant is properly positioned between the vertebral endplates, the distractor, along with its blades, may be removed from the distraction site in the patient.

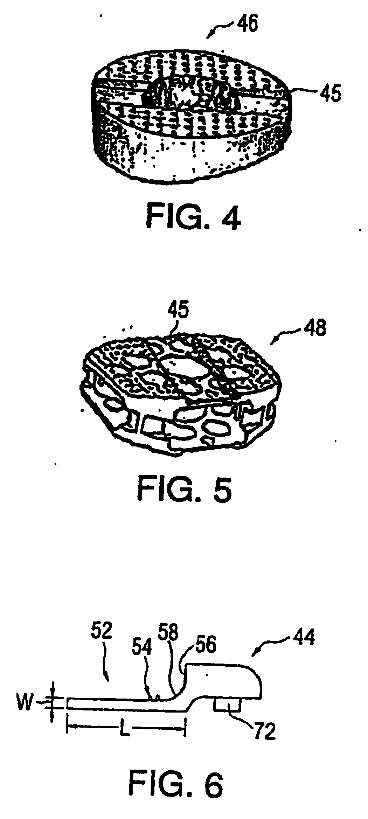

In the embodiment of Figs. 1-3, blades 44a, 44b are provided on jaws 14a, 14b, respectively, to engage the vertebrae to be distracted. In a preferred embodiment, blades 44a, 44b are configured and shaped to correspond to a slot 45 in an implant such as cage 46 of Fig. 4 or femoral ring 48 of Fig. 5. The blades 44a; 44b lie in vertical plane BB shown in Fig. 2. Thus, as the selected implant is moved toward the treatment site with a desired insertion tool, implant contacting surfaces 50a, 50b (Fig. 3) of blades 44a, 44b contact respective slots 45. Preferably, implant contacting surfaces 50a, 50b of blades 44a, 44b are closer together than the point of connection 51a, 51 b of blades 44a, 44b to respective jaws 14a, 14b. Thus, jaws 14a, 14b are sufficiently spaced apart to permit insertion of the thickest dimension of the implant therebetween, yet blades 44a, 44b are closer together to account for the narrower dimension of the implant in the region of slots 45 and thereby to securely grasp the implant via slots 45.

Blades 44 may converge toward each other in a distal direction before actuation of distractor mechanism 30 as may be appreciated with reference to Fig. 1. Thus, upon actuation of distractor mechanism 30 and pivoting apart of jaws 14, blades 44, and particularly outwardly facing distracting surfaces 52a, 52b (positioned to contact the endplates in the treatment site), may be moved into an orientation appropriate for the vertebral region being treated. For example, actuation of distractor mechanism 30 may move distracting surfaces 52a, 52b into a parallel orientation with respect to each other to securely engage endplates which are parallel with respect to each other.

Distracting surfaces 52a, 52b of blades 44a, 44b preferably are shaped to securely engage the vertebrae being treated, particularly the endplates thereof. In a preferred embodiment, distracting surfaces 52a, 52b are configured to securely engage the anterior lip of the vertebral endplates being treated, as shown in the isolated view of blade 44 in Fig. 6. For instance, distracting surfaces 52a, 52b may be provided with vertebral engagers 54a, 54b (Figs. 2 and 6), such as in the form of ridges, which engage the endplates. Transverse engagement walls 56a, 56b (Figs. 2 and 6) may be spaced from vertebral engagers 54a, 54b such that an anterior lip of the vertebral endplates fits therebetween. Engagement surface 58a, 58b (Figs. 2 and 6) between vertebral engagers 54a, 54b and engagement walls 56a, 56b preferably is curved to accommodate the anterior lip of the vertebral endplates as well as to provide a smooth transition from distracting surfaces 52a, 52b to transverse engagement walls 56a, 56b.

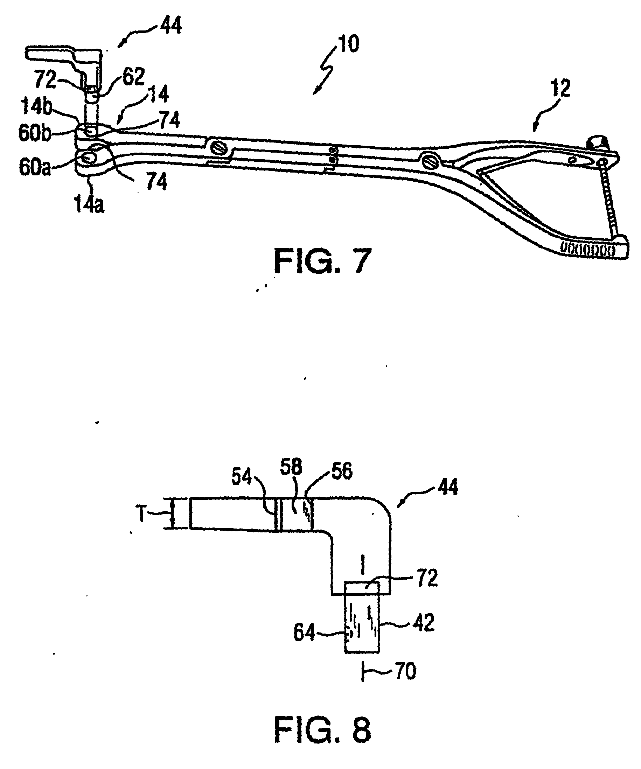

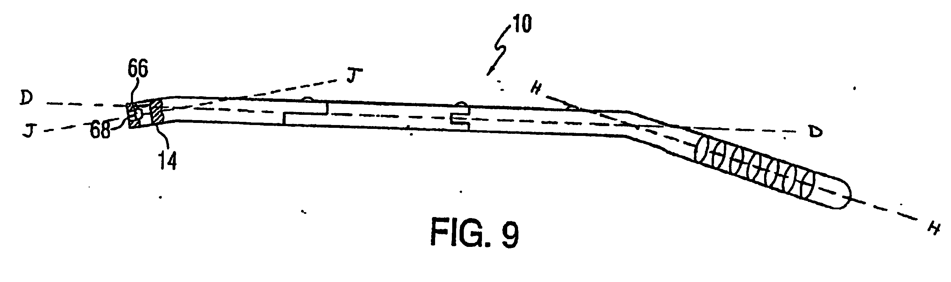

In accordance with the principles of the present invention, the distractor blades preferably are configured to increase versatility of use of the distractor of the present invention. It will be appreciated that distractor 10 preferably is formed from a surgical grade sterilizable metal such that the same distractor may be used for different patients. In order to increase the versatility of distractor 10 and its usefulness for different patients and situations, at least one of blades 44a, 44b may be removably coupled to its respective jaw 14a, 14b, as illustrated in Fig. 7. Thus, in such embodiment, jaws 14a, 14b are provided with a socket 60a, 60b shaped to receive a mounting post 62 of a blade 44, as shown in Figs. 7 and 8. Post 62 may be releasably held within a bore 60 of a jaw 14 in any desired manner. For example, a ball detent attachment may be formed by providing a detent 64 in post 62 (Fig. 8) for matingly receiving a biased engagement ball 66 housed within a transverse bore 68 in jaw 14 (Fig. 9). Blade post 62 preferably is fitted within socket 60 to permit pivotable movement of blade 44 about longitudinal axis 70 of blade post 62. Such pivotable movement facilitates manipulation of blade 44 with respect to the vertebral endplates to ease removal of blade 44 and distractor 10. If desired, in order to limit the range of pivotal motion of blade 44, a stop plate 72 (Figs. 6-8) may be provided on post 62 to fit within range limiting groove 74 (Fig. 7) in jaw 14. Stop plate 72 extends transversely from post 62, as may be appreciated with reference to Fig. 6 and has stop surfaces 76a, 76b engaging respective range limiting surfaces 78a, 78b of range limiting groove 74.

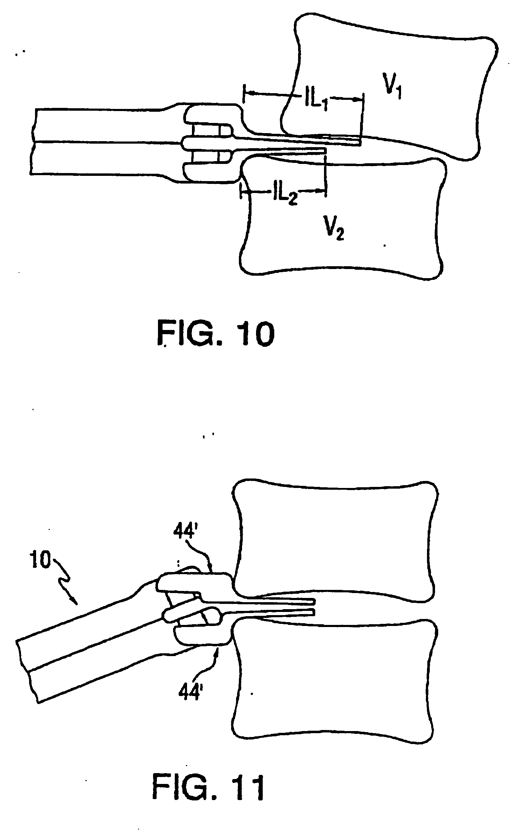

Removable attachment of blades 44 to jaws 14 permits a plurality of differently configured blades to be used with distractor 10 depending on the situation or application. For example, the size of the blade may be selected based on the implant to be inserted, different implants potentially having differently sized slots for receiving a distractor blade. The size of the blade may also be selected depending on the size of the vertebrae being treated or the curvature of the vertebral column. For example, it may be desirable to select blades of different insertion lengths IL1, IL2, as shown in Fig. 10, to account for spondylolisthesis which results in one vertebra V1 being closer to the distractor than the other vertebra V2. Blade selection may also depend on the vertebral region being treated, which may affect the difficulty of the approach. For example, in the pelvic region organs and bony structures may complicate insertion and blades 44 which are angled, such as 20°-30°, with respect to the longitudinal axis 31 of distractor mechanism 30. Such angled blades 44 would permit an angled approach of distractor 10 to avoid bony structures such as the pelvis, but are not part of the present invention as claimed.

The thickness T (Figs. 8) of blades 44a, 44b (the working surface, e.g., distracting surfaces 52a, 52b) is preferably approximately 2-15 mm and most preferably approximately 6-10 mm. The width W (Fig. 6) of blades 44a, 44b is preferably approximately 0,5-4 mm and most preferably approximately 1,5-1,8 mm. The length L (Figs. 6) of blades 44a, 44b is preferably approximately 5-50 mm and most preferably approximately 25-35 mm.

While the foregoing description and drawings represent the preferred embodiments of the present invention, it will be understood that various additions and/or substitutions may be made therein without departing from the scope of the present invention as defined in the accompanying claims. One skilled in the art will appreciate that the invention may be used with many modifications of structure, forms, arrangement, proportions, materials, and components and otherwise, used in the practice of the invention and which are particularly adapted to specific environments and operative requirements, without departing from the principles of the present invention. The presently disclosed embodiments are therefore to be considered in all respects as illustrative and not restrictive, the scope of the invention being indicated by the appended claims, and not limited to the foregoing description.

## Claims

1. A spinal disc space distractor comprising:
A) first and second handles (12);
B) first and second jaws (14) respectively associated with said first and second handles (12);
C) a distractor mechanism (30) coupled between said handles (12) and said jaws (14) such that movement of said handles (12) actuates said distractor mechanism (30) to move said jaws (14) apart; and
D) first and second blades (44) for contacting the surfaces of adjacent vertebrae;
**characterized in that**
D1) said first and second blades (44) are removably couplable directly to said first and second jaws (14). and **in that**
E) the first blade has an insertion length (IL₁) and the second blade has an insertion length (IL₂), and the insertion length of the first blade is different from the insertion length of the second blade.

2. The spinal disc space distractor of claim 1, wherein at least one jaw (14) includes a mounting socket (60) and at least one of said blades (44) is formed with a mounting post (62) shaped for removable insertion within said mounting socket (60).

3. The spinal disc space distractor of claim 1 or 2, wherein each jaw (14) includes a mounting socket (60) and each blade (44) includes a mounting post (62) shaped for removable insertion within said mounting socket (60) of each jaw (14) so that each blade (44) is removably mounted in a mounting socket (60) of a jaw (14).

4. The spinal disc space distractor of claim 2 or 3, wherein each blade (44) is mounted in the mounting socket (60) to selectively position the blades (44) to facilitate separation of the adjacent elements.

5. The spinal disc space distractor of one of claims 2 - 4, wherein said post (62) is releasably held within said socket (60).

6. The spinal disc space distractor of one of the claims 1 - 5, wherein said distal jaw ends (38) are angled with respect to the longitudinal axis (3) of said distractor mechanism (30) at an angle (40) such as 0° to 30°, most preferably 10° relative to the longitudinal axis of the distractor mechanism (30).

7. The spinal disc space distractor of one of the claims 1 - 6, wherein each of the first and second handles (12) is provided with a bend (42), such that proximal handle ends (18) are not in the same plane as distal jaws ends (38).

8. The spinal disc space distractor of one of the claims 1 - 7, further comprising:
a pair of first and second proximal lever arms each having proximal and distal ends; and
a pair of first and second distal lever arms each having proximal and distal ends, wherein:
said first handle (12) is located at said proximal end of said first proximal lever arm; said second handle (12) is located at said proximal end of said second proximal lever arm;
said first jaw (14) is located at said distal end of said first distal lever arm; said second jaw (14) is located at said distal end of said second distal lever arm; one of said pair of proximal lever arms and said pair of distal lever arms is crosswise pivotally coupled; and
the other of said pair of proximal lever arms and said pair of distal lever arms is laterally pivotally coupled.

## Patentansprüche

1. Bandscheibenzwischenraum-Spreizvorrichtung, umfassend:
A) einen ersten und zweiten Handgriff (12);
B) eine erste und zweite Backe (14), die jeweils den genannten ersten und zweiten Handgriffen (12) zugeordnet sind;
C) einen Spreizmechanismus (30) der zwischen den genannten Handgriffen (12) und den genannten Backen (14) derart eingekoppelt ist, dass die Bewegung der genannten Handgriffe (12) den genannten Spreizmechanismus (3) betätigt, um die genannten Backen (14) auseinanderzuspreizen; und
D) eine erste und eine zweite Klinge (44) zur Kontaktaufnahme mit den Oberflächen benachbarter Wirbel,
**dadurch gekennzeichnet, dass**
D1) die genannten ersten und zweiten Klingen (44) abnehmbar an die genannten ersten und zweiten Backen (14) direkt angekoppelt sind;
E) die erste Klinge eine Einführungslänge (IL₁) und die zweite Klinge eine Einführungslänge (IL₂) aufweist, und die Einführungslänge der ersten Klinge sich von der Einführungslänge der zweiten Klinge unterscheidet.

2. Die Bandscheibenzwischenraum-Spreizvorrichtung in Anspruch 1, **dadurch gekennzeichnet, dass** mindestens eine Backe (14) einen Einbausitz (60) umfasst und mindestens eine der genannten Klingen (44) mit einem Eibaustift (62) geformt ist, der für ein abnehmbares Einfügen in den genannten Einbausitz (60) ausgebildet ist.

3. Die Bandscheibenzwischenraum-Spreizvorrichtung in Anspruch 1 oder 2, **dadurch gekennzeichnet, dass** jede Backe (14) einen Einbausitz (60) und jede Klinge (44) einen Einbaustift (62) umfasst, der für ein abnehmbares Einfügen in den genannten Einbausitz (60) jeder Backe (14) geformt ist, sodass jede Klinge (44) in einen Einbausitz (60) einer Backe (14) abnehmbar eingebaut ist.

4. Die Bandscheibenzwischenraum-Spreizvorrichtung in Anspruch 2 oder 3, **dadurch gekennzeichnet, dass** jede Klinge (44) im Einbausitz (60) gelagert ist, um die Klingen (44) wahlweise so einzustellen, dass sie die Trennung der benachbarten Elemente erleichtern.

5. Die Bandscheibenzwischenraum-Spreizvorrichtung in einem der Ansprüche 2 - 4, **dadurch gekennzeichnet, dass** der genannte Stift (62) im genannten Sitz (60) abnehmbar festgehalten ist.

6. Die Bandscheibenzwischenraum-Spreizvorrichtung in einem der Ansprüche 1 - 5, **dadurch gekennzeichnet, dass** die genannten distalen Enden (38) gegenüber der Längsachse (3) des genannten Spreizmechanismus (30) in einem Winkel (40) von 0 - 30°, am besten vorzugsweise 10° gegenüber der Längsachse des Spreizmechanismus (30) abgewinkelt sind.

7. Die Bandscheibenzwischenraum-Spreizvorrichtung in einem der Ansprüche 1 - 6, **dadurch gekennzeichnet, dass** jeder der ersten und zweiten Handgriffe (12) derart mit einer Krümmung versehen ist, dass die proximalen Handgriffsenden (18) nicht in derselben Ebene wie die distalen Backenenden (38) liegen.

8. Die Bandscheibenzwischenraum-Spreizvorrichtung in einem der Ansprüche 1 - 7, weiterhin umfassend:
ein Paar erster und zweiter proximaler Hebelarme, die jeweils ein proximales und distales Ende aufweisen; und
ein Paar erster und zweiter distaler Hebelarme, die jeweils ein proximales und distales Ende aufweisen, wobei
der genannte erste Handgriff (12) am genannten proximalen Ende des genannten ersten proximalen Hebelarmes gelegen ist;
der genannte zweite Handgriff (12) am genannten proximalen Ende des genannten zweiten proximalen Hebelarmes gelegen ist;
die genannte erste Backe (14) am genannten distalen Ende des genannten distalen Hebelarms gelegen ist;
die genannte zweite Backe (14) am genannten distalen Ende des genannten zweiten distalen Hebelarms gelegen ist;
eines der genannten Paare der proximalen Hebelarme und das genannte Paar der distalen Hebelarme gekreuzt drehbar angekoppelt sind; und
das andere der genannten Paare der proximalen Hebelarme und das genannte Paar der distalen Hebelarme seitlich drehbar angekoppelt sind.

## Revendications

1. Écarteur de l'espace intervertébral comprenant :
A) une première et une deuxième poignées (12) ;
B) une première et une deuxième mâchoires (14) associées respectivement auxdites première et deuxième poignées (12) ;
C) un mécanisme écarteur (30) monté entre lesdites poignées (12) et lesdites mâchoires (14) de sorte que le mouvement desdites poignées (12) actionne ledit mécanisme écarteur (30) pour écarter lesdites mâchoires (14) ; et
D) une première et une deuxième lames (44) pour la mise en contact avec les surfaces des vertèbres adjacentes ;
**caractérisé en ce que**
D1) lesdites première et deuxième lames (44) peuvent être couplées de manière amovible directement auxdites première et deuxième mâchoires (14) et **en ce que**
E) la première lame présente une longueur d'insertion (IL₁) et la deuxième lame présente une longueur d'insertion (IL₂), et la longueur d'insertion de la première lame est différente de la longueur d'insertion de la deuxième lame.

2. Écarteur de l'espace intervertébral selon la revendication 1, dans lequel au moins une mâchoire (14) comprend une douille de montage (60) et au moins une desdites lames (44) est formée avec une broche de fixation (62) conçue pour être insérée de manière amovible dans ladite douille de montage (60).

3. Écarteur de l'espace intervertébral selon la revendication 1 ou 2, dans lequel chaque mâchoire (14) comprend une douille de montage (60) et chaque lame (44) comprend une broche de fixation (62) conçue pour être insérée de manière amovible dans ladite douille de montage (60) de chaque mâchoire (14) de sorte que chaque lame (44) est fixée de manière amovible dans une douille de montage (60) sur une mâchoire (14).

4. Écarteur de l'espace intervertébral selon la revendication 2 ou 3, dans lequel chaque lame (44) est fixée dans une douille de montage (60) pour mettre en place les lames (44) de manière sélective afin de faciliter la séparation des éléments adjacents.

5. Écarteur de l'espace intervertébral selon l'une des revendications 2 à 4, dans lequel ladite broche (62) est maintenue temporairement dans ladite douille (60).

6. Écarteur de l'espace intervertébral selon l'une des revendications 1 à 5, dans lequel les extrémités distales (38) desdites mâchoires sont inclinées par rapport à l'axe longitudinal (3) dudit mécanisme écarteur (30) à un angle (40) tel que 0° à 30°, de manière tout particulièrement préférée 10°, par rapport à l'axe longitudinal du mécanisme écarteur (30).

7. Écarteur de l'espace intervertébral selon l'une des revendications 1 à 6, dans lequel chacune des première et deuxième poignées (12) comporte une courbure (42), de sorte que les extrémités proximales (18) des poignées ne sont pas dans le même plan que les extrémités distales (38) des mâchoires.

8. Écarteur de l'espace intervertébral selon l'une des revendications 1 à 7, comprenant en outre :
- une paire d'un premier et d'un deuxième bras de levier proximal, chaque levier présentant des extrémités proximales et distales ; et
- une paire d'un premier et d'un deuxième bras de levier distal, chaque levier présentant des extrémités proximales et distales ;
dans lequel :
- ladite première poignée (12) se trouve à ladite extrémité proximale dudit premier bras de levier proximal ;
- ladite deuxième poignée (12) se trouve à ladite extrémité proximale dudit deuxième bras de levier proximal ;
- ladite première mâchoire (14) se trouve à ladite extrémité distale dudit premier bras de levier distal ;
- ladite deuxième mâchoire (14) se trouve à ladite extrémité distale dudit deuxième bras de levier distal ;
- l'un des bras de levier proximaux de ladite paire et ladite paire de bras de levier distaux sont couplés de manière croisée de façon à pouvoir pivoter ; et
- l'autre paire de bras de levier proximaux et ladite paire de bras de levier distaux sont couplés latéralement de façon à pouvoir pivoter.
